Europäisches Patentamt

European Patent Office

Office européen des brevets

(19)

(11) Veröffentlichungsnummer: **0 245 312**
**B1**

(12) **EUROPÄISCHE PATENTSCHRIFT**

(45) Veröffentlichungstag der Patentschrift: **02.05.90**

(21) Anmeldenummer: **86906253.9**

(22) Anmeldetag: **31.10.86**

(86) Internationale Anmeldenummer:
**PCT/CH86/00151**

(87) Internationale Veröffentlichungsnummer:
**WO 87/02895 21.05.87 Gazette 87/11**

(51) Int. Cl.⁵: **A 61 M 5/24,** A 61 M 5/315

(54) **INJEKTIONSGERÄT.**

(30) Priorität: **08.11.85 CH 4805/85**

(43) Veröffentlichungstag der Anmeldung:
**19.11.87 Patentblatt 87/47**

(45) Bekanntmachung des Hinweises auf die
Patenterteilung:
**02.05.90 Patentblatt 90/18**

(84) Benannte Vertragsstaaten:
**AT CH DE FR GB IT LI NL SE**

(56) Entgegenhaltungen:
**EP-A-0 058 536**
**DE-B-1 070 784**
**US-A-3 481 510**

(73) Patentinhaber: **DISETRONIC AG**
**Brunnmattstrasse 6**
**CH-3400 Burgdorf (CH)**

(72) Erfinder: **MICHEL, Peter**
**Blattnerweg 10**
**CH-3400 Burgdorf (CH)**

(74) Vertreter: **Lusuardi, Werther Giovanni**
**Dr. Lusuardi AG Kreuzbühlstrasse 8**
**CH-8008 Zürich (CH)**

Courier Press, Leamington Spa, England.

EP 0 245 312 B1

**Beschreibung**

Die Erfindung betrifft ein Injektionsgerät gemäss dem Oberbegriff des Patentanspruchs 1.

Ein Gerät dieser Art ist aus der EP—A—0 058 536 bekannt. Bei ihm wird der Kolben der Spritzampulle durch Drehen des Antriebsglieds eines Getriebes vorgeschoben, dessen Abtriebsglied eine am Kolben anliegende Gewindehülse ist. Die Drehbewegung des Antriebsglieds ist durch einen Anschlag begrenzt und eine Kupplung sorgt dafür, dass das Antriebsglied ohne Eingriff mit dem Getriebe vom Anschlag zurückgedreht werden kann. Das Antriebsglied wird jeweils vor dem Injizieren um einen der gewünschten Flüssigkeitsmenge entsprechenden Drehwinkel zurückgedreht und dann beim Injizieren bis zum Anschlag vorwärtsgedreht.

Mit dem bekannten Gerät kann man sich nicht selber in den Arm spritzen, weil man beim Injizieren mit der einen Hand das Gerät halten und mit der anderen Hand das Antriebsglied drehen muss. Beim durch den Anschlag begrenzten Drehen besteht zudem die Gefahr, dass das Gerät und damit auch die Nadel kippt. Besonders nachteilig ist, dass man mit einer Drehbewegung nur eine geringe Flüssigkeitsmenge injizieren kann und zum Injizieren einer grösseren Flüssigkeitsmenge das Antriebsglied — bei eingestochener Nadel — mehrmals hin- und herdrehen müsste.

Der Erfindung liegt die Aufgabe zugrunde, ein Injektionsgerät zu schaffen, an dem eine jeweils zu injizierende, beliebig grosse Flüssigkeitsmenge genau eingestellt bzw. vorgewählt und mit einer einzigen Druckbewegung in Gehäuselängsrichtung injiziert werden kann.

Die erfindungsgemässe Lösung dieser Aufgabe ist Gegenstand des Patentanspruchs 1. Bevorzugte Weiterbildungen der Erfindung sind in den Patentansprüchen 2—13 umschrieben.

Im folgenden werden anhand der Zeichnung Ausführungsbeispiele der Erfindung näher erläutert. Es zeigen:

Figur 1 einen Achsenlängsschnitt durch ein Injektionsgerät, wobei das Getriebeelement in der Ruhelage ist,

Figur 2 einen Achsenlängsschnitt durch den hinteren Teil des Geräts von Figur 1, in grösserem Massstab,

Figur 3 einen Figur 2 entsprechenden Achsenlängsschnitt durch den hinteren Teil des Geräts, wobei das Getriebeelement in der Endlage ist,

Figur 4 einen Querschnitt nach der Linie IV—IV in Figur 3,

Figur 5 einen Querschnitt nach der Linie V—V in Figur 3,

Figur 6 einen Querschnitt nach der Linie VI—VI in Figur 3,

Figur 7 einen Querschnitt nach der Linie VII——VII in Figur 3,

Figur 8 einen Achsenlängsschnitt durch eine bevorzugte Variante des Injektionsgeräts, wobei das Getriebeelement in der Ruhelage ist,

Figur 9 einen Achsenlängsschnitt durch den hinteren Teil des Geräts (ohne Gehäuse) von Figur 8, in grösserem Massstab,

Figur 10 einen Querschnitt nach der Linie X—X in Figur 9, in grösserem Massstab,

Figur 11 eine Seitenansicht eines Teils des Geräts in Blickrichtung XI in Figur 9, in grösserem Massstab, und

Figur 12 einen Querschnitt nach der Linie XII—XII in Figur 9, in grösserem Massstab.

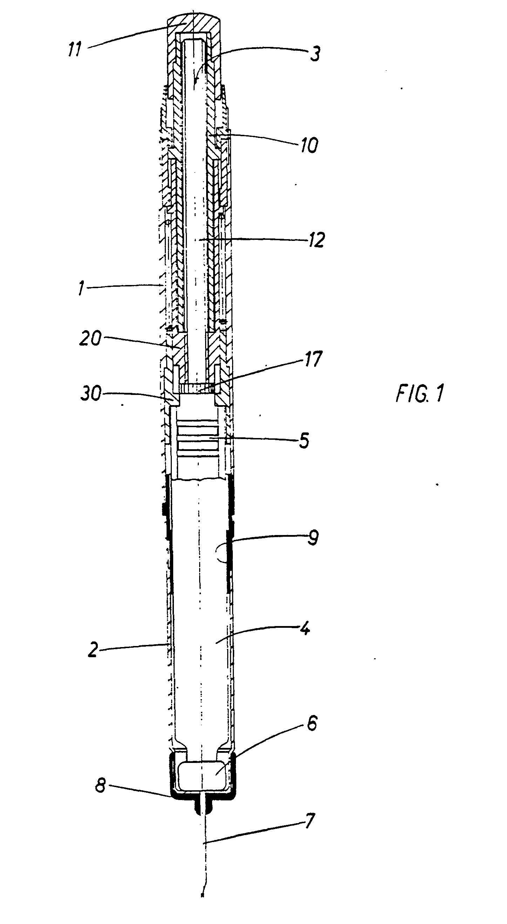

Das in Figur 1—7 dargestellte Injektionsgerät hat zwei hülsenförmige Gehäuseteile 1, 2. Im hinteren (in der Zeichnung oberen) Gehäuseteil 1 ist ein Getriebe 3, im vorderen (in der Zeichnung unteren) Gehäuseteil 2 ist eine Spritzampulle 4 angeordnet. Es handelt sich um eine übliche Spritzampulle (Karpulle), die am einen Ende einen Kolben 5 (ohne Kolbenstange) und am anderen Ende ein durch eine (nicht dargestellte) Membran verschlossenes Auslassstück 6 hat. Der Gehäuseteil 2 hat vorne einen das Auslassstück 6 abstützenden, mit einer Durchführung für die Nadel 7 versehenen Kappenteil 8. Die beiden Gehäuseteile 1 und 2 sind durch eine Gewindemuffe 9 verbunden, wobei der hintere Gehäuseteil 1 mit der Gewindemuffe verklebt ist und der vordere Gehäuseteil 2 zum Auswechseln der Spritzampulle 4 von der Gewindemuffe abgeschraubt werden kann.

Das Antriebsglied des Getriebes 3 besteht aus einer Mitnehmerhülse 10, auf der ein Betätigungskopf 11 sitzt, der zum Drehen und zum nach vorne (unten) Drücken der Mitnehmerhülse 10 dient. Das Abtriebsglied des Getriebes 3 besteht aus einer Gewindestange 12, die drehfest, aber längsverschiebbar in die Mitnehmerhülse 10 eingesetzt ist. Für die drehfeste Lagerung ist die Gewindestange 12 an gegenüberliegenden Seiten 13 und 14 abgeflacht und die gewindelose Bohrung der Mitnehmerhülse 10 ist entsprechend angepasst.

Die Gewindestange 12 hat also zwei ebene, parallele, glatte (gewindelose) Längsflächen 13, 14, an denen sie von den entsprechenden ebenen Innenwandflächen der Mitnehmerhülse 10 mitgenommen wird, und zwei mit dem Gewinde versehene Zylindermantelsegmente 15, 16, die einen Abstand von den beiden glatten (gewindelosen) zylindrischen Innnenwandflächen der Hülse 10 haben. Auf dem vorderen Ende der Gewindestange 12 sitzt eine Scheibe 17, die zum weiter unten beschriebenen Vorschub des Kolbens 5 dient.

Das Getriebeelement, an dem die Gewindestange 12 bewegbar gelagert ist, ist mit 20 bezeichnet. Der vordere Teil 21 des Getriebeelements 20 hat ein Muttergewinde 22, durch das die Gewindestange 12 hindurchgeschraubt ist. Der hintere Teil 23 des Getriebeelements hat eine gewindelose Bohrung, in der die Mitnehmerhülse 10 drehbar gelagert ist. Die Mitnehmerhülse 10 hat einen kreisringförmigen (flanschförmigen) Kragen 25, der auf dem hinteren Ende des Getriebeelementteils 23 aufliegt.

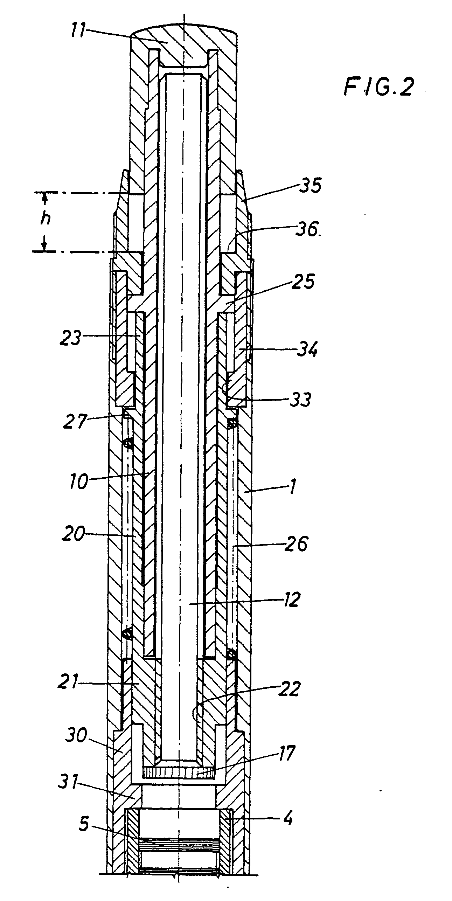

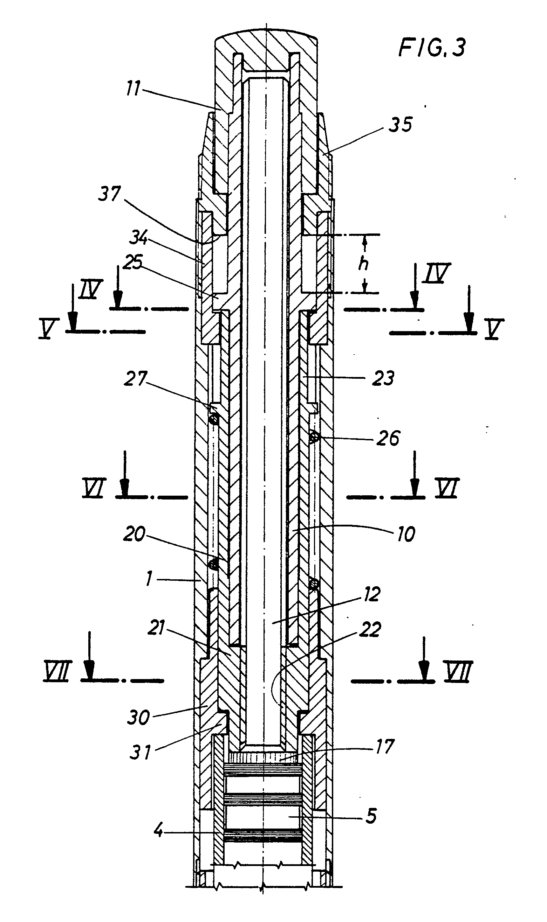

Durch Druck auf den Kopf 11 der Mitnehmerhülse 10 ist das Getriebeelement 20 aus der in Fig. 1 und 2 dargestellten Ruhelage gegen die Kraft einer Druckfeder 26 in die in Fig. 3—7 dargestellte Endlage verschiebbar. Wird der Kopf 11 losgelassen, so wird das Getriebeelement 20 durch die Druckfeder 26 in die Ruhelage zurückgeschoben.

Die Druckfeder 26 ist eine um den Mittelteil des Getriebeelements 20 herumverlaufende Schraubenfeder Sie greift an einem am Element 20 gebildeten Kragen 27 an und ist auf einem hülsenförmigen Stützteil 30 abgestützt, der eine Führung für den vorderen Teil des Getriebeelements 20 bildet und einen radial nach innen ragenden Stützring 31 hat, der auf dem hinteren Rand der Ampulle 4 abgestützt ist. Der vom Kragen 27 nach vorne verlaufende Zylindermantel des Getriebeelements 20 ist dem hülsenförmigen Stützteil 30 mit Spiel angepasst und am vorderen Ende zur Anpassung an den Stützring 31 verengt. Der vom Kragen 27 nach hinten verlaufende Zylindermantel des Getriebeelements 20 ist der Führung 34 mit Spiel angepasst und hat vier in gleichen Abständen voneinander angeordnete Längsnuten 32. In die Nuten 32 greifen vier Längsrippen 33, die am vorderen Teil einer fest im Gehäuseteil 1 verankerten Führung 34 gebildet sind, so dass das Getriebeelement 20 drehfest aber längsverschiebbar am vorderen Teil der Führung 34 gelagert ist. Der hintere Teil der Führung 34 hat eine dem kreisringförmigen Kragen 25 der Mitnehmerhülse 10 angepasste Bohrung und auf seinem hinteren Ende sitzt ein Führungskopf 35. Der vordere Teil des Führungskopfes 35 ist der Mitnehmerhülse 10, der hintere Teil dem Betätigungskopf 11 angepasst. Die ringförmige Schulter- oder Stufenfläche 36 (Fig. 2) am Uebergang zwischen der der Hülse 10 und der dem Kopf 11 angepassten Bohrung des Führungskopfes 35 bildet einen Anschlag für den Betätigungskopf 11. Dieser Anschlag 36 begrenzt die Vorschubbewegung des Betätigungskopfes 11 und damit auch die Verschiebung des Getriebeelements 20. Die in Fig. 3—7 dargestellte Endlage des Getriebeelements 20 ist also erreicht, wenn der Betätigungskopf 11 am Anschlag 36 anschlägt. (Selbstverständlich könnte die Endlage auch durch Anschlagen des Kragens 25 am unteren Teil der Führung 34 oder durch Anschlagen des Getriebeelements 20 am Stützring 31 definiert sein).

In der in Fig. 1 und 2 dargestellten Ruhelage des Getriebeelements 20 ist der Kragen 25 der Mitnehmerhülse 10 durch das von der Feder 26 beaufschlagte Getriebeelement 20 an das vordere Ende 37 (Fig. 3) des Führungskopfes 35 gedrückt. Der Kragen 27 des Getriebeelements 20 hat in der Ruhelage einen geringen Abstand vom vorderen Ende der Führung 34.

Um die jeweils zu injizierende Flüssigkeitsmenge genau vorwählen zu können, ist ein Rastmechanismus vorgesehen, der bei jeder vollen Umdrehung des Betätigungskopfes 11 mehrmals, im vorliegenden Ausführungsbeispiel viermal einrastet, sodass nach jeder Drehung um 90 Grad ein fühlbarer Rastwiderstand überwunden werden muss. Der Rastmechanismus ist durch vier in Winkelabständen von 90 Grad an der hinteren, ringförmigen Endfläche des Getriebeelementteils 23 gebildete, diametral verlaufende Nocken 38 und entsprechende, rinnenförmige Vertiefungen in der dem Getriebeelement 20 zugewandten Ringfläche des Kragens 25 der Mitnehmerhülse 10 gebildet. Die Nocken 38 und die Vertiefungen sind im Querschnitt rund, sodass die Raststellungen ohne Mühe aber doch gegen fühlbaren Rastwiderstand überwunden werden können. Die Feder 26, welche die mit den Nocken 38 versehene Endfläche des Getriebeelements 20 an die mit den Vertiefungen versehene Fläche des Kragens 25 drückt, bildet die Rastfeder des Rastmechanismus.

Die Arbeitsweise des beschriebenen Injektionsgeräts wird nun ausgehend von folgender Ausgangslage beschrieben: Das Getriebe 3 ist in der in Fig. 1 und 2 dargestellten Lage. Dabei sind die Mitnehmerhülse 10 und das Getriebeelement 20 durch die Feder 26 in der Ruhelage gehalten, in welcher der Kragen 25 am Anschlag 37 anliegt. Die Gewindestange 12 ist vollständig zurückgeschraubt, so dass die Scheibe 17 am vorderen Ende des Getriebeelements 20 anliegt. Eine volle Spritzampulle 4 ist in das Gerät eingesetzt. Ihr Kolben 5 sitzt im hinteren Ende der Ampulle und zwar etwas weiter hinten als in Fig. 1 und 2 dargestellt.

Als erstes ist dafür zu sorgen, dass sich die Nadel 7 mit Flüssigkeit füllt. Dazu wird der Kopf 11 nach vorne (unten) gedrückt, bis er am Anschlag 36 anschlägt. Bei diesem Vorschubhub h drückt der Kragen 25 das Getriebeelement 20 mit der in dessen Muttergewinde 22 sitzenden Gewindestange 12 nach vorne gegen die Kraft der Feder 26, wobei die Scheibe 17 am Kolben 5 anschlägt und diesen vorschiebt, so dass eine geringe Flüssigkeitsmenge aus der Nadel 7 austritt. Am Ende des Vorschubhubs h sind alle Teile in der in Fig. 3—7 dargestellten Lage: Die Mitnehmerhülse 10 und das Getriebeelement 20 sind in der durch das Anschlagen des Kopfes 11 am Anschlag 36 definierten Endlage; die Scheibe 17 liegt am Kolben 5 an.

Wenn man den Kopf 11 nun loslässt, drückt die Feder 26 das Getriebeelement 20 und damit auch die in seinem Gewinde 22 sitzende Gewindestange 12 mit der Scheibe 17 sowie die Mitnehmerhülse 10 nach hinten, bis der Kragen 25 am Anschlag 37 anschlägt. Damit sind alle Teile in der in Fig. 1 und 2 dargestellten Lage: Das Getriebeelement 20 und die Mitnehmerhülse 10 sind in ihrer durch die Feder 26 abgestützten Ruhelage und die Scheibe 17 hat einen Abstand h vom Kolben 5, der genau dem Hub h des Getriebeelements 20 zwischen der Ruhe und der Endlage entspricht. Das Gerät ist nun betriebsbereit.

Zum Injizieren einer bestimmten Flüssigkeitsmenge wird zuerst (vor dem Einstechen der Nadel 7) der Kopf 11 bei in der Ruhelage gemäss Fig. 1 und 2 befindlichem Getriebeelement 20 gedreht. Dabei muss nach jeder Drehung um 90 Grad der Rastwiderstand des Rastmechanismus 38 überwunden werden. Jeder Drehung um 90 Grad entspricht eine Flüssigkeitseinheit, sodass die Menge der zu injizierenden Flüssigkeit nach der Anzahl der zu überwindenden Raststellungen bemessen wird. Beim Drehen der Mitnehmerhülse 10 schraubt sich die von ihr mitgenommene Gewindestange 12 durch das Muttergewinde 22 des Getriebeelements 20 nach vorne, wobei sich der Abstand der Scheibe 17 vom Kolben 5 verringert, ohne dass die Scheibe den Kolben berührt. (Der Hub h des Getriebeelements 20 ist grösser bemessen als der für das Injizieren einer maximal zulässigen Flüssigkeitsmenge erforderliche Kolbenweg). Nachdem der Kopf 11 entsprechend der gewünschten Flüssigkeitsmenge gedreht worden ist, wird die Nadel 7 eingestochen und der Kopf 11 bis zum Anschlag 36 gedrückt, wobei das Getriebeelement 20 aus der in Fig. 1 und 2 dargestellten Ruhelage in die in Fig. 3—7 dargestellte Endlage vorgeschoben wird. Während dieses Vorschubhubs h schlägt die Scheibe 17 am Kolben 5 an und schiebt diesen genau soweit vor, wie die Gewindestange 12 durch das vorherige Drehen des Kopfes 11 in bezug auf das Getriebeelement 20 nach vorne gedreht wurde. Nach dem Loslassen des Kopfes 11 drückt die Feder 26 das Getriebeelement 20 wieder in die Ruhelage, wobei die Scheibe 17 sich um den Hub h vom Kolben 5 entfernt. Für die nächste Injektion wird der Kopf 11 wie oben beschrieben wiederum entsprechend der gewünschten Flüssigkeitsmenge gedreht und danach nach unten gedrückt. Um genau die eingestellte Flüssigkeitsmenge zu injizieren, müsste der Vorschubhub grundsätzlich entweder stets bei eingerastetem oder stets bei ausgerastetem Rastmechanismus erfolgen. Die Höhe der Nocken 38 des Rastmechanismus ist aber so klein (z.B. nur zwei Zehntel Millimeter hoch) bemessen, dass die Differenz zwischen dem Vorschubhub bei eingerasteter und ausgerasteter Stellung für die injizierte Flüssigkeitsmenge vernachlässigbar, d.h. kleiner als der Längsweg der Gewindestange bei einer Drehung um 90 Grad ist.

Wenn die Ampulle leer ist, wird der Gehäuseteil 2 von der Gewindemuffe 9 abgeschraubt und die leere Ampulle 4 herausgenommen. Dabei fällt der durch seinen Ring 31 vom hinteren Ende der Ampulle 4 getragene Stützteil 30 nach vorne auf die Gewindemuffe 9. Die Feder 26 wird entlastet und das Getriebeelement 20 nicht mehr an den Kragen 25 gedrückt. Der Kopf 11 kann nun zurückgedreht werden, ohne dass die Rastwiderstände überwunden werden müssten. Das ist wesentlich, weil man vor dem Einsetzen der vollen Ampulle die Gewindestange 12 vollständig durch das Muttergewinde 22 zurückschrauben muss, was sehr viele Umdrehungen erfordert. Nach dem Zurückschrauben der Gewindestange 12 wird der Gehäuseteil 2 mit einer in ihn eingesetzten, neuen, vollen Ampulle 4 wieder an die Gewindemuffe 9 angeschraubt, wobei die Ampulle den Stützteil 30 wieder in die in der Zeichnung dargestellten Lage zurückschiebt. Damit ist die eingangs beschriebene Ausgangslage wieder erreicht.

Zum Schutz der Nadel 7 und des Betätigungskopfes 11 kann vorne und hinten je eine (nicht dargestellte) Kappe auf die Gehäuseteile 1 und 2 gesteckt werden.

Beim in der Zeichnung dargestellten Ausführungsbeispiel hat das vordere Ende der Mitnehmerhülse 10 einen Abstand vom das Muttergewinde 22 aufweisenden, vorderen Teil 21 des Getriebeelements 20. Die Mitnehmerhülse 10 kann aber auch am vorderen Teil 21 anliegen und der Rastmechanismus durch Vorsprünge und Vertiefungen an diesen aneinander anliegenden Flächen gebildet sein, wobei in diesem Fall der Kragen 25 einen Abstand vom hinteren Ende des Getriebeelements 20 hat. Ferner könnte der Rastmechanismus auch durch Vorsprünge und Vertiefungen an den durch die Kraft der Feder 26 aneinander gedrückten Flächen des Kragens 25 der Mitnehmerhülse 10 und des Anschlags 37 gebildet sein. Die Feder 26 könnte statt am Stützteil 30 auch direkt auf dem hinteren Rand der Ampulle 4 abgestützt sein.

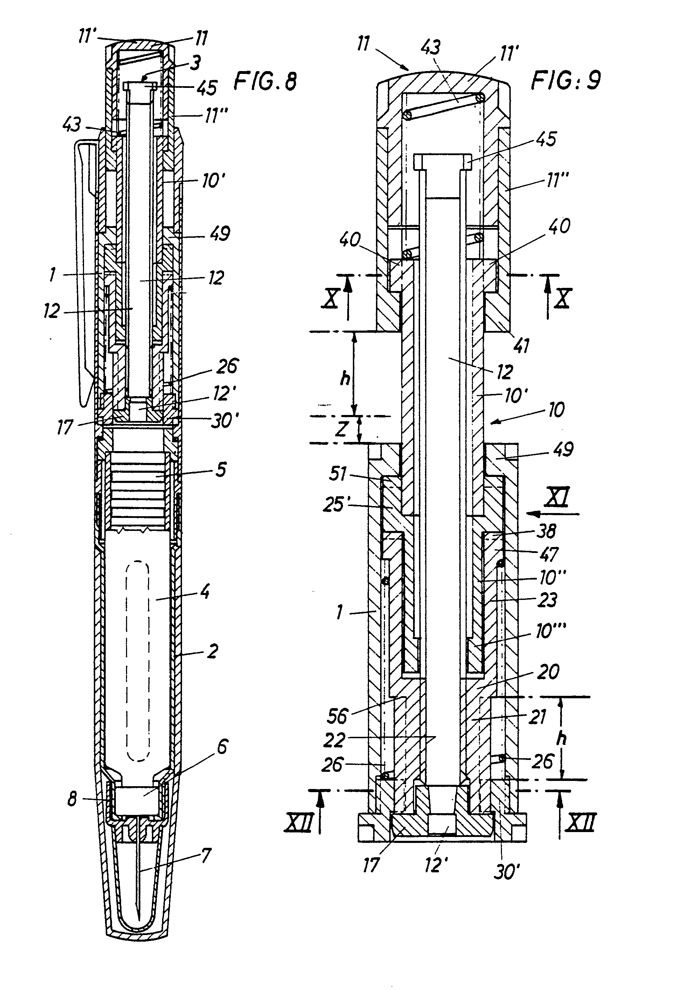

In Figuren 8—12 ist eine bevorzugte Variante des Injektionsgeräts von Figuren 1—7 dargestellt. Die Teile der Variante, welche den im Zusammenhang mit Figuren 1—7 erwähnten Teilen des Injektionsgeräts entsprechen, sind in den Figuren 8—12 mit denselben Bezugszahlen versehen. Die Variante unterscheidet sich wie folgt von der Ausführungsform von Figuren 1—7:

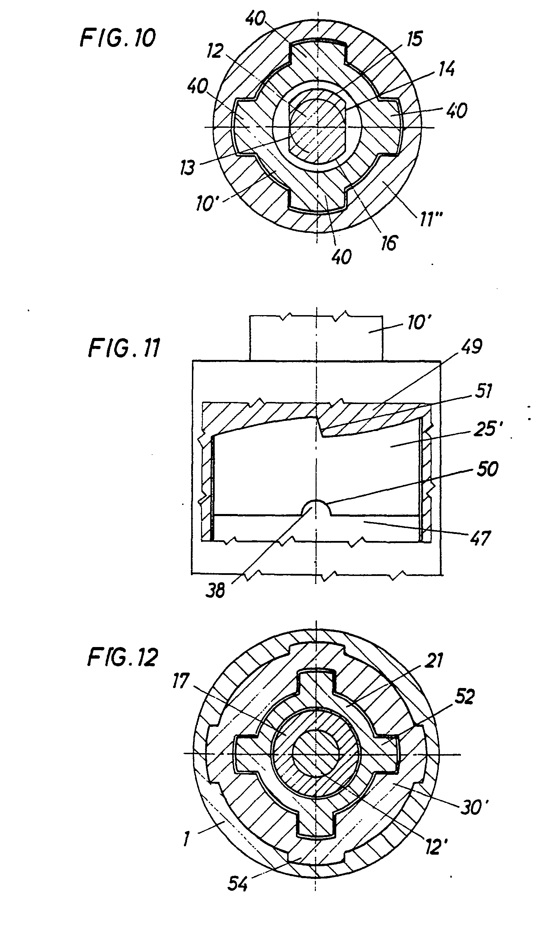

Die das Antriebsglied des Getriebes 3 bildende Mitnehmerhülse 10 besteht aus zwei fest miteinander verbundenen Teilen 10', 10''. Das hintere (obere) Ende des Teils 10'' hat einen radial nach aussen ragenden Kragen 25', in dem das vordere (untere) Ende des Teils 10' sitzt, das vordere Ende hat einen radial nach innen ragenden Ansatz 10''', der die Gewindestange 12 umschliesst. Der aus zwei fest miteinander verbundenen Teilen 11', 11'' bestehende Betätigungskopf 11 sitzt nicht fest auf dem Mitnehmerhülsenteil 10' sondern nur drehfest, aber längsverschiebbar. Dazu hat der Hülsenteil 10' an seinem oberen Ende vier in Gehäuselängsrichtung verlaufende Führungsrippen 40, die in entsprechende, an der Innenwandung des Kopfteils 11'' vorgesehene, in Gehäuselängsrichtung verlaufende Nuten greifen (Fig. 10). Das vordere Ende des Kopfteils 11'' hat einen ringförmig nach innen ragenden, den Mitnehmerhülsenteil 10' umschliessenden Ansatz 41. Im Hohlraum des Kopfes 11 ist eine Druckfeder 43 zwischen den Deckteil des Kopfteils 11' und den oberen Stirnrand des Hülsenteils 10' eingespannt. In der in Fig. 8 und 9 dargestellten Ruhelage ist der Kopf 11 durch seinen Ansatz 41 an den Rippen 40 gegen die Kraft der Feder 43 gehalten.

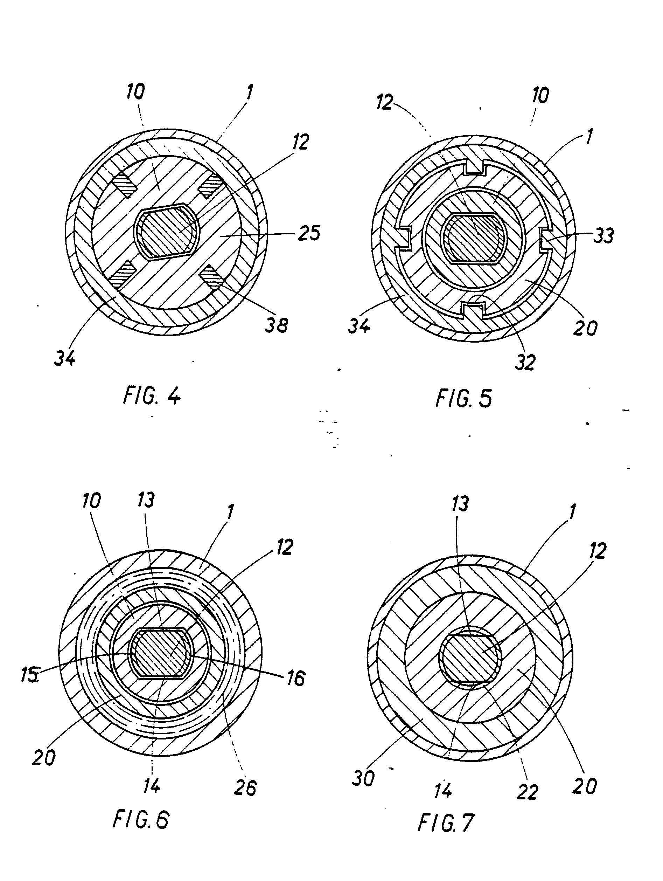

Die das Abtriebsglied des Getriebes 3 bildende Gewindestange 12 ist über ihre ganze Länge an gegenüberliegenden Seiten 13 und 14 abgeflacht, drehfest aber nur im Ansatz 10''' der Mitnehmerhülse 10 gelagert. Nur dieser Ansatz 10''' der Mitnehmerhülse hat also eine der Gewindestange 12 angepasste Innenwandung mit zwei ebenen und zwei zylindrischen (glatten) Innenwandflächen (entsprechend Figuren 4—6). Der übrige Teil der Mitnehmerhülse 10 hat eine zylindrische Bohrung, deren Durchmesser um eine Toleranz grösser ist als der Durchmesser eines am oberen Ende der Gewindestange 12 gebildeten Bunds 45. Wenn die Gewindestange 12 durch das Muttergewinde 22 des vorderen Teils 21 des Getriebeelements 20 geschraubt wird, läuft der Bund 45 durch die Mitnehmerhülse 10 nach vorne (unten), bis er am Ansatz 10''' der Mitnehmerhülse anschlägt.

Das vordere Ende der Gewindestange 12 trägt einen Zapfen 12', auf dem die zum Vorschub des Kolbens 5 dienende Scheibe 17 sitzt.

Die Druckfeder 26 greift an einem am hinteren Ende des Getriebeelementteils 23 gebildeten Kragen 47 an und ist auf einem ortsfest im Gehäuse 1, 2 gehaltenen Stützteil 30' abgestützt. Durch die Feder 26 wird der Kragen 47 gegen den an der Mitnehmerhülse gebildeten Kragen 25' und dieser seinerseits gegen eine am hinteren Ende des Gehäuseteils 1 gebildete, den Mitnehmerhülsenteil 10' umschliessende Schulter 49 gedrückt. Die aneinander anliegenden Flächen der Kragen 47 und 25' haben je vier in Winkelabständen von 90° radial verlaufende Nocken 38 und diesen angepasste rinnenförmige Vertiefungen 50, die unter der Wirkung der Feder 26 einen Rastmechanismus bilden. Der durch die Nocken 38 und Vertiefungen 50 gebildete Rastmechanismus entspricht dem im Zusammenhang mit Figur 1—7 erläuterten. Die aneinander anliegenden Flächen des Kragens 25' und der Schulter 49 haben je ein einander angepasstes, unter der Wirkung der Feder 26 ein Gesperre bildendes Sägezahnprofil 51 (Fig 11). Die Sägezahnprofile 51 an den aneinander angrenzenden Flächen des Kragens 25' und der Schulter 49 haben je vier in Winkelabständen von 90° angeordnete Zähne, wobei die vier Zahnflanken am Kragen 25' in Gehäuselängsrichtung genau über den Vertiefungen 50 des Kragens 25' und die vier Zahnflanken an der Schulter 49 in Gehäuselängsrichtung genau über den Nocken 38 des Getriebeelementteils 23 liegen. Das Getriebeelement 20 ist durch an seinem vorderen Teil 21 vorgesehene Führungsrippen 52 im Stützteil 30' gegen Drehung gesichert verschiebbar; der Stützteil 30' ist ortsfest im Gehäuse 1, 2 verankert und durch Rippen 54 gegen Drehung im Gehäuse gesichert (Fig. 12). Das Getriebeelement 20 ist gegen die Kraft der Feder 26 aus der in Figur 8 und 9 dargestellten Ruhelage um den Hub h nach vorne (unten) verschiebbar, also soweit, bis die am Uebergang zwischen dem vorderen und hinteren Teil 21 und 23 des Getriebeelements 20 gebildete Schulter 56 des Getriebeelements 20 am Stützteil 30' anschlägt.

Die Federkonstante der Feder 43 ist — wie weiter unten näher erläutert — grösser als diejenige der Feder 26, die Feder 43 also härter als die Feder 26, so dass bei Druck auf den Kopf 11 zuerst die Feder 26 zusammengedrückt wird, bis die Schulter 56 am Stützteil 30' anschlägt und erst beim Weiterdrücken des Kopfes die Feder 43 zusammengedrückt wird.

Die Arbeitsweise der Variante des Injektionsgeräts wird nun ausgehend von der in Figuren 8—12 dargestellten Ausgangslage beschrieben, in der die Gewindestange 12 vollständig zurückgeschraubt ist, so dass die Scheibe 17 am vorderen Ende des Getriebeelementteils 21 anliegt, und der Kolben 5 zuhinterst in der gefüllten Spritzampulle 4 sitzt. Vor der ersten Injektion wird nach Entfernen der die Nadel 7 schützenden Schutzkappen der Kopf 11 zum Füllen der Nadel 7 mit Flüssigkeit nach vorne (unten) gedrückt. Da die Feder 43 härter als die Feder 26 ist, bleibt der Kopf 11 dabei in bezug auf die Mitnehmerhülse 10 zunächst in seiner in Figur 8/9 dargestellten Lage. Die auf den Kopf 11 ausgeübte Druckkraft wird durch die Feder 43 auf die Mitnehmerhülse 10 übertragen, die am Kragen 47 des Getriebeelements 20 angreift und dieses mit der in dessen Muttergewinde 22 sitzenden Gewindestange 12 gegen die Kraft der Feder 26 nach vorne (unten) drückt, bis die Schulter 56 am Stützteil 30' anschlägt. Der Kopf 11, die Mitnehmerhülse 10 und die Gewindestange 12 werden also in Bezug auf das Gehäuse 1/2 um den Hub h nach vorne bewegt, wobei die auf dem Zapfen 12' der Gewindestange 12 gelagerte Scheibe 17 während des Hubs h auf den Kolben 5 schlägt und diesen dann auf dem verbleibenden Hubweg etwas nach vorne treibt, so dass eine geringe Menge Flüssigkeit aus der Ampulle 4 durch die Nadel 7 austritt. Nachdem die Schulter 56 am Stützteil 30' angeschlagen hat und somit die Mitnehmerhülse 10 nicht mehr weiter vorgetrieben werden kann, verschiebt sich der Kopf 11 beim Weiterdrücken gegen die Kraft der Feder 43 in Bezug auf die Mitnehmerhülse 10 nach vorne (unten), wobei sich der Ansatz 41 von den Rippen 40 löst und nach einer zusätzlichen Verschiebung z an der Schulter 49 anschlägt. (Wie in Fig. 9 angedeutet, bewegt sich der Kopf 11 also in Bezug auf die Schulter 49 zuerst — mit der Mitnehmerhülse 10 und Gewindestange 12 — um den Hub h, und anschliessend — bei feststehender Mitnehmerhülse 10 und Gewindestange 12 — noch weiter um den zusätzlichen Hub z).

Die Federkonstante der Feder 43 sit einerseits genügend gross gegenüber derjenigen der Feder 26 gewählt, um sicherzustellen, dass der Ansatz 41 des Kopfes 11 zuverlässig an den Rippen 40 gehalten bleibt, bis die gegen die Kraft der Feder 26 und die Reibung des Kolbens 5 in der Ampulle 4 bewegte Mitnehmerhülse 10 mit ihrer Schulter 56 am Stützteil 30, anschlägt. Andererseits ist die Federkonstante der Feder 43 nur gerade so gross gewählt, dass der Patient das Anschlagen der Schulter 56 am Stützteil 30' kaum merkt, d. h. die vom Patienten für die Ueberwindung der Kraft der Feder 26 und der Reibung des Kolbens 5 in der Ampulle 4 notwendige Druckkraft nur unmerklich kleiner ist als die Druckkraft, die für die Bewegung des Kopfes 11 längs des Mitnehmerhülsenteils 10' gegen die Kraft der Feder 43 erforderlich ist. Der Patient erkennt deshalb nicht, dass der Injektionsvorgang am Ende des Hubs h abgeschlossen und der Zusatzhub z zur Injektion nicht notwendig ist. Zweck dieses Zusatzhubs z ist es, sicherzustellen, dass die Schulter 56 der Mitnehmerhülse 10 während einiger Zeit, nämlich der für den Zusatzhub z erforderlichen Zeit, zuverlässig am Stützteil 30' anliegt. Damit wird vermieden, dass der Patient unmittelbar nach dem Anschlagen der Schulter 56 am Stützteil 30' den Kopf 11 loslässt, so dass sich die Scheibe 17 am Ende des Vorschubhubs h sofort wieder vom Kolben 5 löst und die Gefahr besteht, dass der Kolben 5 nicht in der vorgeschobenen Lage bleibt sondern etwas zurückfedert.

Nachdem der Ansatz 41 an der Schulter 49 angeschlagen und deshalb der Kopf 11 nicht weiter vorschiebbar ist, lässt der Patient den Kopf 11 los, worauf die Federn 26 und 43 die Mitnehmerhülse 10 mit der die Scheibe 17 tragenden Gewindestange 12 und den Kopf 11 in die in Fig. 8/9 dargestellte Lage zurückbewegen. (Die Mitnehmerhülse 10 und die Gewindestange 12 bewegen sich dabei um den Hub h, der Kopf 11 um den Hub h + z in bezug auf das Gehäuse 1/2 nach hinten).

Zum Injizieren einer gewünschten Flüssigkeitsmenge dreht der Patient den Kopf 11 jeweils (vor dem Einstechen der Nadel 7) im Uhrzeigersinn um eine der gewünschten Anzahl Flüssigkeitseinheiten entsprechende Anzahl Vierteldrehungen, wobei er für jede Vierteldrehung (Drehung um 90°) eine Raststellung (des Rastmechanismus 38/ 50) zu überwinden hat, die Flüssigkeitsmenge also nach der Anzahl zu überwindender Raststellungen bemessen kann. Beim Drehen des Kopfes 11 und damit auch der drehfest (Führungsrippen 40) mit diesem verbundenen Mitnehmerhülse 10 schraubt sich die vom Mitnehmerhülsenteil 10''' mitgenommene Gewindestange 12 durch das Muttergewinde 22 des Getriebeelements 20 nach vorne, wobei sich der Abstand der Scheibe 17 vom Kolben 5 verringert, ohne dass die Scheibe den Kolben berührt. Eine Drehung im Gegenuhrzeigersinn, durch welche die Gewindestange 12 im Muttergewinde 22 nach hinten geschraubt würde, verhindert das Gesperre 51.

Nachdem die gewünschte Flüssigkeitsmenge durch entsprechendes Drehen des Kopfes 11 vorgewählt ist, wird die Nadel 7 eingestochen und der Kopf bis zum Anschlag (Anschlagen des Ansatzes 41 an der Schulter 49) nach vorne (unten) gedrückt. Dabei wird, wie oben beschrieben, zuerst die Mitnehmerhülse 10 gegen die Kraft der Feder 26 um den Vorschubhub h nach vorne gedrückt, bis sie am Stützteil 30' anschlägt, und anschliessend der Kopf 11 um den zusätzlichen Hub z gegen die Kraft der Feder 43 in Bezug auf die Mitnehmerhülse 10 bewegt. Während des Vorschubhubs h schlägt die Scheibe 17 am Kolben 5 an und schiebt diesen genauso weit vor, wie die Gewindestange 12 durch das vorherige Drehen des Kopfes im Muttergewinde 22 des Getriebeelements 20 nach vorne gedreht wurde. Der zusätzliche Hub z gewährleistet dabei, wie oben erläutert, dass die Mitnehmerhülse 10 mit der Gewindestange 12 während einiger Zeit am Ende des Vorschubhubs h bleibt, und der Kolben 5 während dieser Zeit durch die Scheibe 17 in der richtigen Endlage gehalten wird, so dass er nach der Entlastung nicht mehr zurückweicht.

Wenn nach vielfachem Gebrauch des Geräts die Ampulle 4 nahezu leer ist, nähert sich der Bund 45 der Gewindestange 12 dem Teil 10''' der Mitnehmerhülse. Es tritt dann schliesslich der Fall ein, dass der Patient eine bestimmte Anzahl Flüssigkeitseinheiten injizieren will, der Kopf 11 sich aber nicht mehr um eine entsprechende Anzahl Vierteldrehungen drehen lässt, weil der Bund 45 während der Drehungen am Teil 10''' anschlägt. Damit weiss der Patient, dass er nur noch eine der bereits ausgeführten Anzahl Vierteldrehungen entsprechende und nicht mehr die ganze von ihm gewünschte Flüssigkeitsmenge injizieren kann.

Die Länge der Gewindestange bzw. die Lage des Bunds 45 ist so bemessen, dass der Kolben 5 beim Vorschubhub h der mit ihrem Bund 45 am Teil 10''' der Mitnehmerhülse anliegenden Gewindestange 12 noch zuverlässig vorgeschoben werden kann, ohne an der vorderen Verengung der Ampulle 4 anzuschlagen. Der Patient hat damit die Gewähr, dass die Anzahl Flüssigkeitseinheiten, die er durch Drehen des Kopfes 11 bis zum Anschlagen des Bunds 45 am Teil 10''' eingestellt hat, zuverlässig injiziert werden kann. Und wenn er eine bestimmte Anzahl Flüssigkeitseinheiten injizieren will, den Kopf aber nicht mehr um die entsprechende Anzahl Vierteldrehungen drehen kann, so zeigt ihm dies an, dass er eine neue Ampulle bzw. ein neues Injektionsgerät verwenden muss, um ein zweimaliges Injizieren (zuerst den Rest aus der alten Ampulle und dann die noch verbleibende Differenz aus der neuen Ampulle) zu vermeiden.

Das Injektionsgerät kann als Wegwerfgerät ausgeführt oder die Ampulle 4 kann ausgewechselt werden. Wenn die Ampulle 4 ausgewechselt wird, muss die Gewindestange 12 durch Drehen des Kopfes 11 im Gegenuhrzeigersinn durch das Muttergewinde 22 zurückgeschraubt werden, bis die Scheibe 17 am vorderen Rand des Getriebeelementteils 21 anschlägt. Dazu muss die das Gesperre 51 bildende Sägezahnprofilfläche des Kragens 25' von derjenigen der Schulter 49 gelöst werden. Das kann, wie im Zusammenhang mit Fig. 1—7 beschrieben, dadurch erreicht werden, dass der Stützteil 30' von der Ampulle 4 im Gehäuse 1/2 gehalten ist, so dass er beim Herausnehmen der Ampulle nach unten fällt, wodurch die Feder 26 entlastet und sowohl das Gesperre 51 als auch der Rastmechanismus 38, 50 gelöst wird. Wenn der Stützteil 30, fest im Gehäuse 1/2 verankert ist, kann der Kopf 11 etwas nach vorne (unten) gedrückt werden, so dass sich das Sägezahnprofil des Mitnehmerkragens 25' vom Sägezahnprofil der Schulter 49 löst. Während des Zurückdrehens im Gegenuhrzeigersinn ist dann allerdings der Kopf dauernd gedrückt zu halten und der Rastwiderstand der Rastvorrichtung 38, 50 bei jeder Vierteldrehung zu überwinden.

**Patentansprüche**

1. Injektionsgerät zum Injizieren jeweils wählba-

rer Flüssigkeitsmengen aus einem mit einem Kolben (5) ausgerüsteten Flüssigkeitsbehälter (4), insbesondere einer Spritzampulle (4), mit einem manuell antreibbaren Getriebe (3), das ein in der Vorschubrichtung des Kolbens (5) bewegbares Abtriebsglied (12, 17) hat, und das ein Getriebeelement (20) aufweist, an dem das Abtriebsglied (12, 17) bewegbar gelagert ist, dadurch gekennzeichnet, dass wenigstens das Getriebeelement (20) in der Vorschubrichtung des Kolbens (5) aus einer Ruhelage (Fig. 2; Fig. 9) in eine Endlage (Fig. 3) und wieder zurück in die Ruhelage (Fig. 2; Fig. 9) verschiebbar ist, derart, dass das in der Ruhelage (Fig. 2; Fig. 9) des Getriebeelements (20) vom Kolben (5) distanzierte Abtriebsglied (12, 17) mittels des Getriebes (3) in der Vorschubrichtung entsprechend einem für die jeweils zu injizierende Flüssigkeitsmenge erforderlichen Kolbenweg bewegbar ist, ohne am Kolben (5) anzustossen, und während des Vorschubs (h) des Getriebeelements (20) aus der Ruhein die Endlage am Kolben (5) anstösst und diesen entsprechend dem vorgewählten Kolbenweg vorschiebt.

2. Gerät nach Anspruch 1, dadurch gekennzeichnet, dass das Getriebeelement (20) durch eine Feder (26) in der Ruhelage gehalten und gegen die Federkraft in die Endlage verschiebbar ist.

3. Gerät nach Anspruch 1 oder 2, dadurch gekennzeichnet, dass das Getriebe (3) ein Antriebsglied (10) mit einem am der Injektionsnadel (7) abgewandten Ende des Geräts angeordneten Betätigungskopf (11) hat, mittels dem das Antriebsglied (10) drehbar und zusammen mit dem Getriebeelement (20) aus der Ruhe in die Endlage vorschiebbar ist.

4. Gerät nach den Ansprüchen 2 und 3, dadurch gekennzeichnet, dass der Betätigungskopf (11) drehfest, aber längsverschiebbar auf dem Antriebsglied (10) gelagert (40) und gegen die Kraft einer entgegengesetzt zur Vorschubrichtung auf ihn wirkenden, zweiten Feder (43) an einem Teil (40) des Antriebsglieds (10) abgestützt ist, und dass die Federkonstante der zweiten Feder (43) grösser als diejenige der ersten Feder (26) ist, so dass beim Drücken auf den Betätigungskopf (11) dieser zuerst gemeinsam mit dem Antriebsglied (10), dem Getriebeelement (20) und dem Abtriebsglied (12, 17) gegen die Kraft der ersten Feder (26) aus der Ruhe in die Endlage vorgeschoben wird, und unmittelbar anschliessend beim Weiterdrücken auf den Betätigungskopf (11) dieser gegen die Kraft der zweiten Feder (43) in Bezug auf das Antriebsglied (10) vorgeschoben wird, wobei das Antriebsglied (10), das Getriebeelement (20) und das Abtriebsglied (12, 17) in der Endlage bleiben.

5. Gerät nach den Ansprüchen 2 und 3 oder 4, dadurch gekennzeichnet, dass das Getriebeelement (20) durch die Kraft der ersten Feder (26) an das Antriebsglied (10, 25) gedrückt ist und seine Ruhelage durch einen Anschlag (37; 49) für das Antriebsglied (10, 25) bestimmt ist.

6. Gerät nach einem der Ansprüche 1-5, dadurch gekennzeichnet, dass das Antriebsglied des Getriebes (3) durch eine mittels eines Betätigungsorgans (11) oder direkt manuell drehbare Mitnehmerhülse (10) und das Abtriebsglied durch eine Gewindestange (12) gebildet ist, die durch die Mitnehmerhülse (10) längsverschiebbar und mindestens in deren vorderem Teil (10''') drehfest gehalten ist, und dass das Getriebeelement (20) ein die Gewindestange (12) aufnehmendes Muttergewinde (22) hat und durch eine Führung (33, 34; 30') gegen Drehung gesichert (32; 52) ist.

7. Gerät nach Anspruch 6, dadurch gekennzeichnet, dass die Gewindestange (12) am hinteren Ende einen Bund (45) aufweist, die Innenwandung der Mitnehmerhülse (10) nur im vordersten Teil (10''') zur drehfesten Halterung der Gewindestange (12) ausgebildet und im übrigen Teil dem Bund (12) mit Spiel angepasst- ist, und dass die Länge der Gewindestange (12) bzw. Lage des Bunds (45) an der Gewindestange (12) so in bezug auf die Länge des Flüssigkeitsbehälters (4) bemessen ist, dass dessen Kolben (5) beim Vorschubhub (h) der mit ihrem Bund (45) am vordersten Teil (10''') der Mitnehmerhülse (10) angeschlagenen Gewindestange (12) noch zuverlässig vorschiebbar ist, ohne an der Vorderseite des Flüssigkeitsbehälters (4) anzuschlagen.

8. Gerät nach Anspruch 6 oder 7, dadurch gekennzeichnet, dass der in Vorschubrichtung vordere Teil (21) des Getriebeelements (20) das Muttergewinde (22) aufweist und der hintere Teil (23) eine gewindelose Bohrung hat, in der die Mitnehmerhülse (10) drehbar gelagert ist, und dass die Mitnehmerhülse (10) und das Getriebeelement (20) aneinander abgestützt (25, 23; 25', 47) sind.

9. Gerät nach einem der Ansprüche 1 bis 8, dadurch gekennzeichnet, dass das Getriebe (3) ein drehbares Antriebsglied (10, 11) und einen Rastmechanismus (38) aufweist, der bei jeder vollen Umdrehung des Antriebsglieds (10, 11) ein oder mehrmals einrastet, so dass beim manuellen Drehen des Antriebsglieds (10, 11) jeweils ein fühlbarer Rastwiderstand überwunden werden muss und sich die Menge der jeweils zu injizierenden Flüssigkeit nach der Anzahl der zu überwindenden Raststellungen bemessen lässt.

10. Gerät nach den Ansprüchen 5 und 9, dadurch gekennzeichnet, dass der Rastmechanismus durch Vorsprünge (38) und Vertiefungen (50) an den durch die erste Feder (26) aneinander gedrückten Flächen des Antriebsglieds (10, 25; 10, 25') und des Getriebeelements (20, 23; 20, 47) oder Anschlags (37; 49) gebildet ist.

11. Gerät nach einem der Ansprüche 3-10, dadurch gekennzeichnet, dass das Getriebe (3) ein selbsttätiges Gesperre (51) aufweist, das die Drehbewegung des Antriebsglieds (10, 11) in dem Drehsinn freigibt, in dem das Abtriebsglied (12, 17) in Vorschubrichtung antreibbar ist, und im entgegengesetzten Drehsinn sperrt.

12. Gerät nach den Ansprüchen 10 und 11, dadurch gekennzeichnet, dass die erste Feder (26) zwischen einem fest im Gehäuse (1, 2) oder an der Rückseite der Spritzampulle (4) abgestützten Stützteil (30') und einem am Getriebeelement (20) gebildeten Vorsprung (56) eingespannt ist, und von den beiden durch die Kraft der ersten Feder (26) aneinander gedrückten Ringflächenpaaren des Antriebsglieds (10, 25') und des Getriebeelements (20, 47) sowie des Antriebsglieds (10, 25') und des Anschlags (49) das eine Ringflächenpaar (25', 49) das Gesperre bildende, sägezahnartige Umfangsprofile (51) und das andere Ringflächenpaar (25', 47) die Vorsprünge (38) und Vertiefungen (50) des Rastmechanismus aufweist.

13. Gerät nach einem der Ansprüche 9 bis 12, mit auswechselbarem Flüssigkeitsbehälter (4), dadurch gekennzeichnet, dass die den Rastmechanismus (38; 38, 50) und/oder das Gesperre (51) belastende erste Feder (26) am Flüssigkeitsbehälter oder an einem am Flüssigkeitsbehälter (4) abgestützten Stützteil (30) abgestützt ist, der beim Herausnehmen des Flüssigkeitsbehälters (4) aus dem Gerät in eine Lage gleitet, in der die erste Feder (26) den Rastmechanismus und/oder das Gesperre (51) nicht mehr oder nur noch geringfügig belastet, so dass das Antriebsglied (10, 11) nach Herausnehmen des Flüssigkeitsbehälters (4) ohne Ueberwindung eines Rastwiderstands und/ oder in der Sperrichtung des Gesperres (51) drehbar ist.

## Revendications

1. Instrument d'injection destiné à injecter des quantités de liquide éventuellement sélectives à partir d'un récipient de liquide (4) muni d'un piston (5), notamment d'une ampoule à injecter (4), avec un mécanisme (3) actionnable manuellement, qui comporte un élément mené (12, 17) mobile dans le sens d'avance du piston (5), sur lequel est logé de façon mobile l'élément mené (12, 17), caractérisé en ce qu'au moins l'élément de mécanisme (20) est déplaçable dans le sens d'avance du piston (5) à partir d'une position de repos (fig. 2; fig. 9) jusque dans une position d'extrémité (fig. 3) et de nouveau en retour dans la position de repos (fig. 2; fig. 9) de telle manière que dans la position de repos (fig. 2; fig. 9) de l'élément de mécanisme (20), l'élément mené (12, 17) séparé par le piston (5), est mobile au moyen du mécanisme (3) dans la direction d'avance à concurrence de la course de piston nécessaire pour la quantité de liquide à injecter selon le cas, sans venir heurter le piston (5), et pendant l'avance (h) de l'élément de mécanisme (20) depuis la position de repos jusqu'à la position d'extrémité vient s'appuyer contre le piston (5) et fait avancer celui-ci en fonction de la course de piston présélectionnée.

2. Dispositif selon la revendication 1, caractérisé en ce que l'élément de mécanisme (20) est maintenu par un ressort (26) dans la position de repos et peut être déplacé en opposition à la force du ressort jusque dans la position d'extrémité.

3. Instrument selon les revendications 1 ou 2, caractérisé en ce que le mécanisme (3) comporte un élément d'entraînement (10) avec une tête d'actionnement (11) agencée sur le côté détourné de l'aiguille d'injection (7) de l'instrument, au moyen duquel l'élément d'entraînement (10) est rotatif et conjointement avec l'élément de mécanisme (20) peut être déplacé depuis la position de repos jusqu'à la position d'extrémité.

4. Instrument selon les revendications 2 et 3, caractérisé en ce que la tête d'actionnement (11) est montée (14) de façon fixe en rotation mais mobile longitudinalement sur l'élément d'entraînement (10) et s'appuie contre la force d'un deuxième ressort (43) agissant sur lui en opposition au sens de déplacement sur une partie (40) de l'élément d'entraînement (10), et en ce que la constante de ressort du deuxième ressort (43) est supérieure à celle du premier ressort (26) de sorte que lors de la pression exercée sur la tête d'actionnement (11) celle-ci est d'abord déplacée conjointement avec l'élément d'entraînement (10), l'élément de mécanisme (20) et l'élément mené (12, 17) en opposition à la force du premier ressort depuis la position de repos jusqu'à la position d'extrémité, et aussitôt après lors de la poursuite de la compression sur la tête d'actionnement (11), celle-ci est déplacée en opposition à la force du deuxième ressort (43) par rapport à l'élément d'entraînement (10), moyennant quoi l'élément d'entraînement (10), l'élément de mécanisme (20) et l'élément mené (12, 17) demeurent dans la position d'extrémité.

5. Instrument selon les revendications 2 et 3 ou 4, caractérisé en ce que l'élément de mécanisme (20) est comprimé par la force du premier ressort (26) sur l'élément d'entraînement (10, 25) et sa position de repos est déterminée par une butée (37; 49) pour l'élément d'entraînement (10, 25).

6. Instrument selon l'une des revendications 1-5, caractérisé en ce que l'élément d'entraînement du mécanisme (3) est formé par une gaine d'entraînement (10) rotative au moyen d'un organe d'actionnement (11) ou directement de façon manuelle et l'élément mené est constitué par une tige filetée (12), pouvant se déplacer longitudinalement à travers la gaine d'entraînement (10) et se trouve maintenu de façon résistante en rotation au moins dans sa partie avant (10'''), en ce que l'élément du mécanisme (20) comporte un filet femelle (22) recevant la tige filetée (12) et en ce qu'il est assujetti grâce à un guide (33, 34; 30') contre la rotation (32; 52).

7. Instrument selon la revendication 6, caractérisé en ce que la tige filetée comporte sur l'extrémité arrière une collerette (45), la paroi intérieure de la gaine d'entraînement (10) n'est formée que dans la partie la plus avant (10''') pour le maintien en position résistante à la torsion de la tige filetée (12) et dont la partie restante est adaptée à la collerette (12) avec un jeu en ce que la longueur de la tige filetée (12) ou la position de la collerette (45) sur la tige filetée (12) est dimensionnée de façon relative à la longueur du récipient de liquide (4), et en ce que son piston (5), lors de la course d'avance (h) de la tige filetée (12) attaquée par sa collerette (45) sur la partie la plus avant (10''') de la gaine d'entraînement (10), peut encore se déplacer en toute sécurité sans venir heurter le côté avant du récipient de liquide (4).

8. Instrument selon les revendications 6 ou 7, caractérisé en ce que la partie avant (21) dans le sens d'avance de l'élément d'entraînement (20) comporte le filet femelle (22) et la partie arrière (23) comporte un alésage sans filetage, dans lequel est logée de façon rotative la gaine d'entraînement (10), et en ce que cette gaine et l'élément de mécanisme (20) sont appuyés l'un sur l'autre (25, 23; 25', 47).

9. Dispositif selon l'une des revendications 1 à 8, caractérisé en ce que le mécanisme (3) comporte un élément d'entraînement rotatif (10, 11) et un mécanisme d'encliquetage (38) qui, pour chaque tour complet de l'élément d'entraînement (10, 11), s'encliquette une ou plusieurs fois, de sorte que lorsque l'on fait tourner manuellement l'élément d'entraînement (10) on doit vaincre une résistance d'encliquetage sensible, ce qui permet de doser selon le cas la quantité du liquide injectée en fonction du nombre de positions d'encliquetage à dépasser.

10. Instrument selon les revendications 5 et 9, caractérisé en ce que le mécanisme d'encliquetage est formé par des saillies (38) et des dépressions (50) sur les surfaces comprimées entre elles par le premier ressort (26) de l'élément d'entraînement (10, 25; 10, 25') et de l'élément de mécanisme (20, 23; 20, 47) ou de la butée (37; 49).

11. Dispositif selon l'une des revendications 3-10, caractérisé en ce que le mécanisme (3) comporte un cliquet automatique (51) qui autorise le mouvement de rotation de l'élément d'entraînement (10, 11) dans le sens de rotation dans lequel l'élément mené (12, 17) peut être entraîné dans le sens d'avance, et interdit le mouvement de rotation dans le sens de rotation opposé.

12. Dispositif selon les revendications 10 et 11, caractérisé en ce que le premier ressort (26) est tendu entre une partie d'appui (30') supportée solidement dans le boîtier (1, 2) ou sur le côté arrière de l'ampoule d'injection (4) et une saillie (56) formée sur l'élément de mécanisme (20), et par les deux paires de surfaces annulaires appuyées l'une contre l'autre par la force du premier ressort (26), de l'élément d'entraînement (10, 25') et de l'élément du mécanisme (20, 47) ainsi que de l'élément d'entraînement (10, 25') et de la butée (49) qui comporte une paire de surfaces annulaires (25', 49) les profilés périphériques (51) en dents de scie formant le cliquet et l'autre paire de surfaces annulaires (25', 47) les saillies (38) et des pressions (50) du mécanisme d'encliquetage.

13. Instrument selon l'une des revendications 9 à 12, avec des récipients de liquide échangeables (4), caractérisé en ce que le premier ressort (26) chargeant le mécanisme d'encliquetage (38; 38, 50) et/ou le cliquet (51) vient s'appuyer sur un récipient de liquide (4), ou sur une partie d'appui appuyée contre le récipient (30), lequel, lors de l'extraction du récipient de liquide (4), glisse hors de l'instrument dans une position dans laquelle le premier ressort (26) ne charge plus le mécanisme d'encliquetage et/ou le cliquet (51) ou ne le charge que dans des proportions infimes, de sorte que l'élément d'entraînement (10, 11), après extraction du récipient de liquide (4), peut être mis en rotation sans surmonter une résistance d'encliquetage et/ou dans la direction interdite du cliquet (51).

**Claims**

1. An injection instrument for injecting selectable quantities of liquid from a liquid container (4) equipped with a piston (5), the container being in particular an injection ampoule (4) with a manually drivable transmission (3) having an output member (12, 17) adapted for movement in the direction of feed of the piston (5), and which comprises a gear element (20), characterised in that at least the gear element (20) is displaceable in the direction of feed of the piston (5) out of a rest position (Fig. 2; Fig. 9) into an extreme position (Fig. 3) and back again into the rest position (Fig. 2; Fig. 9), so that the output member (12, 17), which in the rest position (Fig. 2; Fig. 9) of the gear element (20) is spaced apart from the piston (5), can be moved by the gear mechanism (3) in the direction of feed and in accordance with a piston travel which is required for whatever quantity of fluid has to be injected, without abutting the piston (5) and in that during forward movement (h) of the gear element (20), the output member (12, 17) moves out of the rest position and into the extreme position, striking the piston (5) and pushing this latter forwards in accordance with the preselected piston travel.

2. An instrument according to Claim 1, characterised in that the gear element (20) is retained in the rest position by a spring (26) and can be displaced into the extreme position against the force of the spring.

3. An instrument according to Claim 1 or 2, characterised in that the gear mechanism (3) has a drive member (10) with an actuating head (11) disposed at the end of the instrument which is remote from the injection needle (7) and by means of which the drive member (10) is rotatable and can be moved forwards together with the gear element (20) out of the rest position and into the extreme position.

4. An instrument according to Claims 2 and 3, characterised in that the actuating head (11) is rotationally rigid but longitudinally displaceable on the drive member (10) (at 40) and is braced on a part (40) of the drive member (10) against the force of a second spring (43) acting on it in opposition to the direction of feed, and in that the spring constant of the second spring (43) is greater than that of the first spring (26), so that when the actuating head (11) is pressed, this latter is first advanced jointly with the drive member (10), the gear element (20) and the output member (12, 17) and against the force of the first spring (26) out of the rest position and into the extreme position and, immediately afterwards, as further pressure is applied to the actuating head (11), this latter is advanced against the force of the second spring (43) and is moved in respect of the drive member (10), this latter, the gear element (20) and the output member (12, 17) remaining in the extreme position.

5. An instrument according to Claims 2 and 3 or 4, characterised in that the gear element (20) is pressed by the force of the first spring (26) against the drive member (10, 25), its rest position being determined by an abutment (37, 49) for the drive member (10, 25).

6. An instrument according to one of Claims 1 to 5, characterised in that the drive member of the gear mechanism (3) is constituted by a driver sleeve (10) adapted for direct manual rotation or by means of an actuating member (11), the output member being a screw-threaded rod (12) which is adapted for longitudinal displacement by the driver sleeve (10) and is held rotationally rigidly at least in the forward part (10′′′), and in that the gear element (20) has a female screw thread (22) accommodating the screw-threaded rod (12) and is secured (32, 52) against rotation by a guide (33, 34; 30′).

7. An instrument according to Claim 6, characterised in that the screw-threaded rod (12) has a shoulder (45) at the rear end and in that the inner walls of the driver sleeve (10) are only in the foremost part (10′′′) constructed for rotationally rigid supporting of the threaded rod (12) and in that the rest of it is adapted to the collar (12) (sic!) with clearance and in that the length of the threaded rod (12) or the position of the collar (45) on the threaded rod (12) is so dimensioned in respect of the length of the liquids container (4) that at the forwards movement (h) of the threaded rod (12) the collar (45) on which abuts the foremost part (10′′′) of the driver sleeve (10) the piston (5) can still be advanced reliably without striking the front face of the liquids container (4).

8. An instrument according to Claim 6 or 7, characterised in that the (in the direction of feed) front part (21) of the gear element (20) carries the female thread (22) while the rear part (23) has a non-threaded bore in which the driver sleeve (10) is rotatably fitted and in that the driver sleeve (10) and the gear element (20) are braced on each other (25, 23; 25′, 47).

9. An instrument according to one of Claims 1 to 8, characterised in that the gear mechanism (3) comprises a rotatable drive member (10, 11) and a catch mechanism (38) which engages once or a plurality of times at each complete rotation of the drive member (10, 11), so that upon manual rotation of the drive member (10, 11) it is always necessary to overcome a perceptible resistance from the catch mechanism, the quantity of whichever fluid is to be injected being calculated according to the number of catch positions which have to be overcome.

10. An instrument according to Claims 5 and 9, characterised in that the catch mechanism is formed by projections (38) and depressions (50) on those faces of the drive (10, 25; 10, 25′) and of the gear element (20, 23; 20, 47) or abutment (37, 49) which are pressed on one another by the first spring (26).

11. An instrument according to one of Claims 3 to 10, characterised in that the gear mechanism (3) has an automatic lock (51) which releases the rotary movement of the drive member (10, 11) in the direction of rotation in which the output member (12, 17) can be driven in the direction of feed and locks it in the opposite direction of rotation.

12. An instrument according to Claims 10 and 11, characterised in that the first spring (26) is clamped between a bracing member (30′) which is rigid in the housing (1, 2) or which is braced against the back of the injection ampoule (4) and a projection (56) formed on the gear element (20) and in that of the two pairs of annular faces on the drive member (10, 25′) and on the gear element (20, 47) as well as on the drive member (10, 25′) and the abutment (49) which are pressed against each other by the force of the first spring (26), one pair of annular faces (25′, 49) comprises sawtooth-like peripheral profiles (51) which form the locking means while the other pair of annular faces (25′, 47) comprise the projections (38) and depressions (50) of the catch mechanism.

13. An instrument according to one of Claims 9 to 12, with an interchangeable fluids container (4), characterised in that the first spring (26) biasing the catch mechanism (38; 38, 50) and/or the locking mechanism (51) is braced on the liquids container or on a supporting part (30) which is braced on the liquids container (4) and which, when the liquids container (4) is removed from the instrument, slides into a position in which the first spring (26) no longer or only slightly loads the catch mechanism and/or the locking mechanism (51), so that after the liquids container (4) has been removed, the drive member (10, 11) is rotatable without overcoming any resistance from the catch mechanism and/or is rotatable in the locking direction of the locking mechanism (51).

FIG. 1

FIG.2

EP 0 245 312 B1

FIG.3

FIG. 4

FIG. 5

FIG. 6

FIG. 7

FIG. 8

FIG. 9

FIG.10

FIG.11

FIG.12